Europäisches Patentamt

European Patent Office

Office européen des brevets

(11) Publication number: **0 101 441**
**B1**

## (12) EUROPEAN PATENT SPECIFICATION

(45) Date of publication of patent specification: **14.01.87**

(21) Application number: **82901187.3**

(22) Date of filing: **12.02.82**

(86) International application number:
**PCT/US82/00182**

(87) International publication number:
**WO 83/02723 18.08.83 Gazette 83/19**

(51) Int. Cl.⁴: **A 61 K 31/70,** A 61 K 31/66,
A 61 K 31/52

(54) ANTI-VIRAL COMPOSITIONS.

(43) Date of publication of application:
**29.02.84 Bulletin 84/09**

(45) Publication of the grant of the patent:
**14.01.87 Bulletin 87/03**

(84) Designated Contracting States:
**AT BE CH DE FR GB LI LU NL SE**

(56) References cited:
US-A-3 767 795
US-A-4 199 574
US-A-4 215 113
US-A-4 294 831
US-A-4 323 573

Shipkowitz et al. Applied Microbiology, vol. 26, pp. 264-267 (1973)

Gillen, Synthesis and Properties of Novel Nucleoside and Nucleotide Analogues, Thesis Department of Chemistry McGill University, Montreal, Quebec, Canada, (April 1980), pp. 18,19 & 82

Declercq et al, Chemical Abstracts, vol. 91, no. 133836c (1979)

(73) Proprietor: ens BIO LOGICALS INC.
20, Victoria Street
Toronto Ontario, M5C 2N8 (CA)

(72) Inventor: SMITH, Kendall O.
133 Trillium
San Antonio, TX 78213 (US)

(74) Representative: Perry, Robert Edward et al
GILL JENNINGS & EVERY 53-64 Chancery Lane
London WC2A 1HN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to pharmaceutical compositions, and more particularly to pharmaceutical compositions useful for treatment of viral infections such as herpes infections in mammals.

Herpes simplex virus (HSV) infections are widespread in human populations, and pose a particularly difficult health problem. Genital herpes poses a serious health threat to women, in particular. Pregnant women with active genital herpes infections at the time of delivery have a 50—50 chance of passing it on to their babies. The American Academy of Pediatrics states that 60% of those babies born with HSV infections will die, and half of the survivors will suffer severe damage to the brain, nervous system and eyes ("Pediatrics" *66*, 147—9, 1980). It has also been proposed that HSV2 may have a role in the onset of cervical cancer. There has been observed an association between sexual intercourse and cervical cancer, which may be explained by transmission of HSV-2.

Unlike other sexually transmitted diseases such as gonorrhea, syphilis and nongonococcal urethritis, there is currently no cure for herpes infections. Many of the drugs currently in clinical use may not be effective in reducing the severity or the duration of the symptoms. Even after the symptoms disappear, herpes virus tends to remain dormant in nerve tissue, only to be reactivated at a later date to an active phase of infection, causing lesions ("cold sores") and other symptoms to recur. A drug can be considered effective if it diminishes the severity of the lesions, allows for more rapid healing, extends the period between recurrences of herpes infections or stops recurrences altogether.

Herpes simplex virus is one member of the family "Herpetoviridae"; other members of this family which infect humans are varicella-zoster, cytomegalovirus and Epstein-Barr virus. The family also includes various members which attack animals. For example, there are three types of equine herpesvirus, a swine herpesvirus, a canine herpesvirus and a feline herpesvirus, among others.

As with all viruses, herpes virus invades healthy host cells on which it relies to provide its needs for replication. Herpes viruses (code for) some of the enzymes they need for replication, instead of relying completely on the host cell for all their needs. Hence, herpes viruses are subject to selective inhibition by certain drugs that interfere specifically with viral enzymes.

A variety of drugs have been proposed and tested for treatment of HSV infections. For example, U.S. Patent 4,199,574 discloses a wide variety of compounds said to be useful in such treatments, extensive testing of one of which, i.e. acycloguanosine (ACG) or acyclovir, 9 - [2 - hydroxyethoxymethyl]guanine, has been reported in the literature, with sometimes promising results. Another drug which has been explored is 5-iododeoxyuridine (IDU), but this has been reported to be effective only against herpes infections of the eyes. It also has undesirable side-effects, associated with toxicity to normal cells. Adenine arabinoside (ara-A), phosphonoformic acid (PFA), phosphonoacetic acid (PAA), 2-deoxy-D-glucose (2DG) and 5 - (2 - halovinyl) - 2' - deoxyuridines, as exemplified by bromovinyl-deoxyuridine (BVDU) and its iodo-analogue (IVDU), are other drugs which have some demonstrated activity against human herpes viruses.

US—A—4508898 discloses further compounds having activity against herpes simplex viruses, which show promise of providing effective treatment of diseases caused by these viruses. The most promising of these drugs is 9 - [[2 - hydroxy - 1 - (hydroxymethyl)ethoxy]methyl]guanine, hereinafter sometimes referred to as G*.

According to the present invention, a therapeutic composition comprises, in combination, a first active component which is G* and a second active component selected from PAA and salts thereof, PFA and salts thereof, ACG and 5 - (2 - halovinyl) - 2' - deoxyuridines (e.g. BVDU or IVDU) and in which the ratio of the first active component to the second active component is from 1:200 to 200:1 on a molar basis.

Compositions of the invention have activity against herpes virus, whilst having no significant effect on normal host cells at anti-virally effective dosage levels. The combinations are much more effective than could be predicted from consideration of the activities of the compounds individually, indicating that there is some synergic effect. Combinations of G* and PAA, and of G* and PFA, are outstanding.

Whilst it is not intended that the present invention should in any way be limited to any specific theory as to the basis of the results obtained, the following hypothesis is offered for better and fuller understanding and explanation of the invention. Part of the explanation of the outstanding results obtainable with the combinations of the invention may, it is felt, lie in the specific modes of action by which the respective active components interfere with viral replication in the infected host cells, or the general metabolism of such infected host cells.

The aim of viral chemotherapy is to suppress viral replication in infected cells, or to damage the metabolism of infected cells, without substantially damaging uninfected cells. Thus, inhibition of viral replication may depend upon blocking one or more virus-specific metabolic steps in infected cells, or "poisoning" the infected cells only. The mechanism of antiviral action of acycloguanosine, for example, appear to be interaction with the herpes virus-specific thymidine kinase (TK) resulting in phosphorylation of the acycloguanosine. The resulting phosphorylated compound then inhibits the action of the herpes virus-specific DNA-polymerase (DP), thereby preventing replication of the viral DNA and replication of the virus itself. Cellular TK and DP enzymes are only slightly affected by the drug. Thus, acycloguanosine has relatively low toxicity for uninfected cells, and relatively high antiviral activity. Bromovinyl-deoxyuridine

**0 101 441**

(BVDU) appears to have an essentially similar mode of action, although it may in fact only be active primarily against HSV-1 and not HSV-2.

Phosphonoacetic acid (PAA) and phosphonoformic acid (PFA) appear to have only a single mechanism of action, namely interference with viral DNA polymerase, not viral thymidine kinase.

In contrast, the mode of action of G* appears to be different from that of the other drugs, and, in fact, unique. It seems that it inhibits the activity of the viral thymidine kinase enzyme, but is not involved in the activity of virus-specific DNA-polymerase. Thus the drug G* appears to be specific for viral TK and, in addition, does not require virus-specific DP for its activity. This unique anti-viral mode of action may be the reason why it is synergistic with other drugs; also, G* appears to be able to inhibit HSV mutants lacking the ability to induce DP (i.e. DP-mutants), which mutants are highly resistant to acycloguanosine, BVDU and PAA.

The preferred compositions of the present invention have been found to be extremely active against a wide representative variety of strains of HSV, both types I and II. Whilst there is some variation in the degree of activity against different HSV strains, as would be expected, there is high activity against a wide variety thereof, and synergy between the active ingredients G* and either PAA or PFA in all cases. The compositions are also similarly active against equine herpesvirus of various types, and swine herpesvirus (pseudorabies virus).

Nearly every HSV strain produces virus particles which are partially resistant to each of the drugs mentioned above. For example, if one examines a typical titration curve which shows the effect of varying drug concentrations upon HSV plaque formations (the viral plaque titration method of Roizman and Roane referred to in more detail below), the curve is sigmoid, i.e. there are a few viral plaques which emerge in the presence of drug concentration which readily suppresses other plaques. It is these partially drug-resistant virus plaques which can sometimes be suppressed by a second drug possibly having a mode of action different from the first drug. The result may be synergistic action between the first and second drugs, as set out below.

The relative amounts of the drugs in the compositions according to the invention can be varied over wide limits. The optimum amount of each drug varies according to the selection of drug, the nature of the formulation in which it is to be applied, the type and strain of HSV to be treated, and the severity and location of the infection, among other factors. Appropriate relative amounts are found in the 1:200—200:1 molar range, most likely within the 25:1—1:25 molar range. The synergistic action appears to be exhibited throughout the entire range of relative proportions of the drugs. In practice, however, it is most preferred to use approximately equal proportions of the two drugs, e.g. in the 5:1—1:5 molar range.

For administration to patients, the compositions of the invention may be applied topically as ointment, cream or powder, parenterally, interthecally, as nose drops, eye drops or as an aerosol for inhalation, again depending upon the nature and location of the infection to be treated. Suitable dosage rates are in accordance with those known and established as effective with the individual drugs of the composition. Effective unit doses for administration of the compositions interthecally or parenterally are suitably in the range from about 0.1—100 mg of each drug in the chosen combinations, per kg mammal body weight, most suitably in the 0.5—20 mg per kg and most preferably about 5 mg per kg, on the basis of a dosage administered from 2—4 times daily. It is preferred to treat the infection with relatively large doses of the combination of drugs at the outset, so as to limit the chances of development of resistant viral strains in the infection.

For topical administration, ointments or creams in conventional inert bases (e.g. petrolatum) can be formulated, in the known way. An amount from about 0.1—5 weight per cent of the each drug, preferably from about 0.5—2 weight per cent of each drug, provides a suitable concentration in an ointment or cream, for topical administration 1—4 times per day. Such topically applied formulations are effectively holding a reservoir of the active drugs against the infected site, so that the concentrations of drugs in the formulations are not critical, provided of course that a dosage level harmful to surrounding skin areas is not used. With PAA, topical formulation with drug concentrations in excess of 100 mM have been reported as skin irritant, whereas such amounts of PFA do not appear harmful.

Detailed description of specific preferred embodiments

The invention is further illustrated in the following specific experimental results and examples.

Example 1

Human fetal fibroblasts (HFF) derived from fetal tissues were used in this study. Cells were grown and maintained in Basal Medium Eagle (BME) supplemented with 0.112% sodium bicarbonate, 2 mML-glutamine, 2 mg% Neomycin and 20% (vol/vol) calf serum.

A variety of HSV strains was used in the tests, as set out in the Tables presented below.

HSV-1-Patton is a well-known, standard strain, readily available from culture collections. It is known to be mid-range in terms of its sensitivity to known drugs. HSV-2-EL 1, HSV-2-EL 3, HSV-2-EL 4, HSV-2-EL 6, HSV-1-EL 11 and HSV-1-227 are recent clinical isolates.

A viral plaque titration method (Roizman and Roane, 1961) was used to determine the titer of the HSV strain. Tissue culture dishes (35 by 10 mm, Corning) were seeded with cells and used for assays when they were approximately 75% monolayer. Volumes (0.2 ml) of logarithmic dilutions of the virus strain were

3

inoculated onto each of two tissue culture dishes and adsorbed for 1 hr with intermittent shaking, the inoculum was removed and 2 ml of 20% BME containing 0.5% human immune serum globulin was added. After a 48 h incubation period at 36°C in a 5% $CO_2$ atmosphere, the overlay medium was removed, and the cell sheets were stained with a 0.05% aqueous crystal violet solution. The plaque numbers were counted with the aid of a Nikon profile projector which magnified the dishes 10×. Duplicate results were averaged, and the number of plaque-forming units (PFU) was calculated. The virus titre is thus expressed as a number of plaque forming units to be seen after growth under these conditions.

As antiviral drugs in these experiments, there were used acycloguanosine (ACG), phosphonoacetic acid (PAA), bromovinyl deoxyuridine (BVDU), phosphonoformic acid (PFA) and 9 - [[2 - hydroxy - 1 - (hydroxymethyl) - ethoxy]methyl]guanine (G*). Stock solutions, 3 mg/ml, of each drug were prepared as follows, kept at −20°C, and appropriate dilutions thereof were made in 20% BME just before usage:

G*-3 mg dissolved in 0.1 ml distilled water and 0.9 ml 20% BME added;

ACG—3 mg dissolved in 0.1 ml of 0.1N NaOH and brought to volume with 20% BME;

PAA—3 mg dissolved in 1 ml of 20% BME

PFA—3 mg dissolved in 1 ml of 20% BME

BVDU—3 mg dissolved in 1 ml of 20% BME.

In order to make comparisons between the activities of the various drugs and combinations, experiments were conducted by plaque titration to determine for each drug its viral ED-50 (i.e. the concentration thereof which inhibits HSV plaque formation by 50% in the HFF cells, as compared with cell cultures in the absence of the drug). For this purpose, tissue culture dishes (35 by 10 mm) with HFF cell monolayers at 75% confluence were inoculated with approximately 50 plaque-forming units of virus per 0.2 ml, and the virus was allowed to adsorb for 1 hr with intermittent shaking. After removal of the inoculum, 2 ml of 20% BME with 0.5% immune globulin and threefold dilutions of the appropriate drug were added to duplicate dishes. One set of dishes received no drug and was later used for the "no-drug control". After a 48 h incubation period at 36°C in a 5% $CO_2$ atmosphere, the overlay medium was removed, the cells were stained as described above, and plaques were counted. After averaging the counts of duplicate plates, the fraction of plaques emerging in the presence of each drug dilution was calculated as a percent of the control. Each drug titration was plotted on semilogarithmic graph paper, with the number of plaques per dish as the vertical axis and the drug concentration as the horizontal axis. The viral $ED_{50}$ was calculated as that amount of drug per ml of overlay medium that inhibited plaque numbers by 50%, compared to a control (no drug), and this was read off the graphic plot of the data.

Three combinations of the drugs were then tested for activity by viral plaque titration, at concentrations which were multiples of the $ED_{50}$ of the individual drugs. When G*, BVDU or ACG was present, a concentration ten times that of its $ED_{50}$ was used in the combination. This was intended to ensure a concentration sufficiently high to limit the numbers of partially drug-resistant virus plaques to a small fraction, in respect of each drug of the combination. In respect of PAA, a concentration of five times the $ED_{50}$ was used, to avoid cell toxicity problems. The drugs were individually plaque-titrated at the same concentrations, for comparison purposes.

The results are given in the first Table headed "HSV-1-PATTON". The column headed "VT" gives the virus titre, expressed in plaque-forming units (PFU) per ml, $\times 10^5$; of course, the lower the figure, the greater the inhibitory, anti-viral effect of the tested drug (D). The column headed "FTR" gives the fold titre reduction which is the ratio of virus titre for the control, i.e. no drug, to that of the given test. (‡ indicates that this titration did not give a linear dose-response curve; the given value was calculated from the mean plaque counts at $10^{-2-5}$ and $10^{-3}$ virus dilutions).

The results show that all three drug combinations exhibit a degree of synergy. However, the G*-PAA combination is truly outstanding, its activity being of a totally different order of magnitude from that of either of the individual ingredients.

Example 2

In this Example, combinations of G* and PFA were tested, by plaque titration methods, for activity against three different herpes virus strains in HFF cells, using procedures previously described. A stock solution of PFA was made up ahead of time, by dissolving 3 mg PFA in 1 ml of 20% BME, and stored at −20°C. It was appropriately diluted in 20% BME just before usage. The results are given in three Tables identifying the three different strains, i.e. the second Table headed "HSV-1-PATTON", the Table headed "HSV-2-EL4", and the Table headed "HSV-2-EL6".

The results show that the combination of drugs has synergic activity against all three HSV strains. The apparently relatively low activity of G* alone against HSV-1-Patton, by comparison with the results of Example 1, is probably the result of virus samples of different activity. Each strain will have a different $ED_{50}$ with respect to a given drug.

Example 3

The experiments described in Examples 1 and 2 were essentially repeated, but using G* and PAA alone and in combination against samples of HFF cells each infected with one of six HSV strains. The results of plaque titration methods are given in the Tables headed "HSV-2-EL1" and "HSV-2-EL3", the second Table

4

headed "HSV-2-EL4", the second Table headed "HSV-2-EL6", and the Tables headed "HSV-1-EL11" and "HSV-1-227".

The results show that the two drugs exhibit synergy against all of the tested HSV strain in HFF cells. The relative concentrations of the drugs in the combinations in these experiments, as in the other Examples, are multiples of their $ED_{50}$ values. These relative amounts are appropriate to testing *in vitro*, and to demonstrate the synergy, but are illustrative only. Relative amounts in combinations for use *in vivo*, and in practical administration for HSV treatments, are as previously discussed, and may not bear close relationship to the proportions in the specific Examples.

### HSV-1-PATTON

| D | D (µg/ml) | VT | FTR |
|---|---|---|---|
| — | 0 | 147 | 1 |
| G* | 1.2 | 1.1 | 130 |
| BVDU | 0.11 | 2.1 | 70 |
| G*+BVDU | 1.2+0.11 | 0.3 | 490 |
| ACG | 2.0 | 4.7 | 31 |
| G*+ACG | 1.2+2.0 | 0.5‡ | 290 |
| PAA | 250 | 3.2 | 50 |
| G*+PAA | 1.2+250 | 0.016 | 9,200 |

### HSV-1-PATTON

| D | D (µg/ml) | VT | FTR |
|---|---|---|---|
| — | 0 | 3400 | 1 |
| G* | 0.7 | 140 | 24 |
| PFA | 80 | 750 | 4.5 |
| G*+PFA | 0.7+80 | <0.05 | >68,000 |

### HSV-2-EL4

| D | D (µg/ml) | VT | FTR |
|---|---|---|---|
| — | 0 | 750 | 1 |
| G* | 3.0 | 21 | 36 |
| PFA | 80 | 0.28 | 2,700 |
| G*+PFA | 3.0+80 | 0.005 | 150,000 |

### HSV-2-EL6

| D | D (µg/ml) | VT | FTR |
|---|---|---|---|
| — | 0 | 150 | 1 |
| G* | 0.7 | 40 | 3.8 |
| PFA | 80 | 48 | 3.1 |
| G*+PFA | 0.7+80 | <0.005 | >30,000 |

### HSV-2-EL1

| D | D (µg/ml) | VT | FTR |
|---|---|---|---|
| — | 0 | 170 | 1 |
| G* | 2.0 | 8.9 | 19 |
| PAA | 76 | 0.34 | 500 |
| G*+PAA | 2.0+76 | <0.005 | >34,000 |

### HSV-2-EL3

| D | D (µg/ml) | VT | FTR |
|---|---|---|---|
| — | 0 | 77 | 1 |
| G* | 2.0 | 10 | 7.7 |
| PAA | 82 | 0.36 | 210 |
| G*+PAA | 2.0+82 | <0.005 | >15,400 |

### HSV-2-EL4

| D | D (µg/ml) | VT | FTR |
|---|---|---|---|
| — | 0 | 750 | 1 |
| G* | 2.0 | 5.9 | 130 |
| PAA | 90 | 0.10 | 7,500 |
| G*+PAA | 2.0+90 | <0.005 | >150,000 |

### HSV-2-EL6

| D | D (µg/ml) | VT | FTR |
|---|---|---|---|
| — | 0 | 180 | 1 |
| G* | 0.6 | 0.90 | 200 |
| PAA | 90 | 31.0 | 5.8 |
| G*+PAA | 0.6+90 | <0.005 | >36,000 |

### HSV-1-EL11

| D | D (µg/ml) | VT | FTR |
|---|---|---|---|
| — | 0 | 8.0 | 1 |
| G* | 0.4 | 0.37 | 22 |
| PAA | 25 | 2.8 | 2.9 |
| G*+PAA | 0.4+25 | <0.005 | >1,600 |

### HSV-1-227

| D | D (µg/ml) | VT | FTR |
|---|---|---|---|
| — | 0 | 28 | 1 |
| G* | 2.0 | 0.25 | 110 |
| PAA | 80 | 1.1 | 26 |
| G*+PAA | 2.0+80 | <0.005 | >930 |

**Claims for all Designating States: BE, CH, DE, FR, GB, LI, LU, NL, SE**

1. A therapeutic composition having activity against herpes viral infections, which comprises, in combination, a first active component which is 9 - [[2 - hydroxy - 1 - (hydroxymethyl)ethoxy]methyl]guanine, and a second active component selected from phosphonoacetic acid and salts thereof, phosphonoformic acid and salts thereof, acycloguanosine and 5 - (2 - halovinyl) - 2' - deoxyuridines, and in which the ratio of the first active component to the second active component is from 1:200 to 200:1, on a molar basis.

2. A composition according to claim 1, wherein said second active component is phosphonoacetic acid or a salt thereof.

3. A composition according to claim 1, wherein said second active component is phosphonoformic acid or a salt thereof.

4. A composition according to claim 1, wherein said second active component is acycloguanoguanosine.

5. A composition according to claim 1, wherein said second active component is bromovinyl-deoxyuridine.

6. A composition according to claim 1, wherein said second active component is iodovinyl-deoxyuridine.

7. A composition according to any preceding claim, wherein the molar ratio of the active components is from 5:1 to 1:5.

8. Use of two active components for the manufacture of a medicament for the treatment of herpes viral infections, in which the active components are combined in a ratio of from 1:200 to 200:1, on a molar basis, and in which the first active component is 9 - [[2 - hydroxy - 1 - (hydroxymethyl)ethoxy]methyl]guanine and the second active component is selected from phosphonoacetic acid and salts thereof, phosphonoformic acid and salts thereof, acycloguanosine and 5 - (2 - halovinyl) - 2' - deoxyuridines.

9. Use according to claim 8, wherein the second active component is as defined in any of claims 2 to 6.

10. Use according to claim 8 or claim 9, wherein the molar ratio of the active components is from 5:1 to 1:5.

**Claims for the Contracting State: AT**

1. A method for preparing a therapeutic composition having activity against herpes viral infections, which comprises combining two active components in a ratio of from 1:200 to 200:1, on a molar basis, in which the first active component is 9 - [[2 - hydroxy - 1 - (hydroxymethyl)ethoxy]methyl]guanine and the second active component is selected from phosphonoacetic acid and salts thereof, phosphonoformic acid and salts thereof, acycloguanosine and 5 - (2 - halovinyl) - 2' - deoxyuridines.

2. A method according to claim 1, wherein said second active component is phosphonoacetic acid or a salt thereof.

3. A method according to claim 1, wherein said second active component is phosphonoformic acid or a salt thereof.

4. A method according to claim 1, wherein said second active component is acycloguanoguanosine.

5. A method according to claim 1, wherein said second active component is bromovinyl-deoxyuridine.

6. A method according to claim 1, wherein said second active component is iodovinyl-deoxyuridine.

7. A method according to any preceding claim, wherein the molar ratio of the active components is from 5:1 to 1:5.

8. Use of two active components for the manufacture of a medicament for the treatment of herpes viral infections, in which the active components are combined in a ratio of from 1:200 to 200:1, on a molar basis, and in which the first active component is 9 - [[2 - hydroxy - 1 - (hydroxymethyl)ethoxy]methyl]guanine and the second active component is selected from phosphonoacetic acid and salts thereof, phosphonoformic acid and salts thereof, acycloguanosine and 5 - (2 - halovinyl) - 2' - deoxyuridines.

9. Use according to claim 8, wherein the second active component is as defined in any of claims 2 to 6.

10. Use according to claim 8 or claim 9, wherein the molar ratio of the active components is from 5:1 to 1:5.

**Patentansprüche für die Vertragstaaten: BE, CH, DE, FR, GB, LI, LU, NL und SE**

1. Therapeutische Zusammensetzung mit Wirksamkeit gegen Herpesvirusinfektionen, welche in Kombination eine erste aktive Komponente, die 9 - [[2 - Hydroxy - 1 - (hydroxymethyl) - äthoxy] - methyl] - guanin ist, und eine zweite aktive Komponente ausgewählt aus Phosphonoessigsäure und Salzen hievon, Phosphonoameisensäure und Salzen hievon, Acycloguanosin und 5 - (2 - Halogenvinyl) - 2' - desoxyuridinen umfaßt, und in der das Verhältnis der ersten aktiven Komponente zur zweiten aktiven Komponente 1:200 bis 200:1, auf Molbasis, beträgt.

2. Zusammensetzung nach Anspruch 1, worin die zweite aktive Komponente Phosphonoessigsäure oder ein Salz hievon ist.

3. Zusammensetzung nach Anspruch 1, worin die zweite aktive Komponente Phosphonoameisensäure oder ein Salz hievon ist.

**0 101 441**

4. Zusammensetzung nach Anspruch 1, worin die zweite aktive Komponente Acycloguanoguanosin ist.

5. Zusammensetzung nach Anspruch 1, worin die zweite aktive Komponente Bromvinyldesoxyuridin ist.

6. Zusammensetzung nach Anspruch 1, worin die zweite aktive Komponente Jodvinyldesoxyuridin ist.

7. Zusammensetzung nach einem vorhergehenden Anspruch, worin das Molverhältnis der aktiven Komponenten 5:1 bis 1:5 beträgt.

8. Verwendung von zwei aktiven Komponenten zur Herstellung eines Medikaments zur Behandlung von Herpesvirusinfektionen, wobei die aktiven Komponenten in einem Verhältnis von 1:200 bis 200:1, auf Molbasis, vereinigt werden und wobei die erste aktive Komponente 9 - [[2 - Hydroxy - 1 - (hydroxymethyl) - äthoxy] - methyl] - guanin ist und die zweite aktive Komponente ausgewählt ist aus Phosphonoessigsäure und Salzen hievon, Phosphonoameisensäure und Salzen hievon, Acycloguanosin und 5 - (2 - Halogenvinyl) - 2' - desoxyuridinen.

9. Verwendung nach Anspruch 8, wobei die zweite aktive Komponente wie in einem der Ansprüche 2 bis 6 definiert ist.

10. Verwendung nach Anspruch 8 oder Anspruch 9, worin das Molverhältnis der aktiven Komponenten 5:1 bis 1:5 beträgt.


**Patentansprüche für den Vertragstaat: AT**

1. Verfahren zur Herstellung einer therapeutischen Zusammensetzung mit Wirksamkeit gegen Herpesvirusinfektionen, welches das Vereinigen von zwei aktiven Komponente in einem Verhältnis von 1:200 bis 200:1, auf Molbasis, umfaßt, wobei die erste aktive Komponente 9 - [[2 - Hydroxy - 1 - (hydroxymethyl) - äthoxy] - methyl] - guanin ist und die zweite aktive Komponente ausgewählt ist aus Phosphonessigsäure und Salzen hievon, Phosphonoameisensäure und Salzen hievon, Acycloguanosin und 5 - (2 - Halogenvinyl) - 2' - desoxyuridinen.

2. Verfahren nach Anspruch 1, worin die zweite aktive Komponente Phosphonoessigsäure oder ein Salz hievon ist.

3. Verfahren nach Anspruch 1, worin die zweite aktive Komponente Phosphonoameisensäure oder ein Salz hievon ist.

4. Verfahren nach Anspruch 1, worin die zweite aktive Komponente Acycloguanoguanosin ist.

5. Verfahren nach Anspruch 1, worin die zweite aktive Komponente Bromvinyldesoxyuridin ist.

6. Verfahren nach Anspruch 1, worin die zweite aktive Komponente Jodvinyldesoxyuridin ist.

7. Verfahren nach einem vorhergehenden Anspruch, worin das Molverhältnis der aktiven Komponenten 5:1 bis 1:5 beträgt.

8. Verwendung von zwei aktiven Komponente zur Herstellung eines Medikaments zur Behandlung von Herpesvirusinfektionen, wobei die aktiven Komponenten in einem Verhältnis von 1:200 bis 200:1, auf Molbasis, vereinigt werden und wobei die erste aktive Komponente 9 - [[2 - Hydroxy - 1 - (hydroxymethyl) - äthoxy] - methyl] - guanin ist und die zweite aktive Komponente ausgewählt ist aus Phosphonoessigsäure und Salzen hievon, Phosphonoameisensäure und Salzen hievon, Acycloguanosin und 5 - (2 - Halogenvinyl) - 2' - desoxyuridinen.

9. Verwendung nach Anspruch 8, wobei die zweite aktive Komponente wie in einem der Ansprüche 2 bis 6 definiert ist.

10. Verwendung nach Anspruch 8 oder Anspruch 9, worin das Molverhältnis der aktiven Komponenten 5:1 bis 1:5 beträgt.


**Revendications pour les Etats contractants: BE, CH, DE, FR, GB, LI, LU, NL, SE**

1. Une composition thérapeutique douée d'activité contre les infections à virus d'herpès, qui comprend en combinaison un premier composant actif qui est la 9 - [[2 - hydroxy - 1 - (hydroxyméthyl)éthoxy]méthyl]guanine, et un second composant actif choisi parmi l'acide phosphonoacétique et ses sels, l'acide phosphonoformique et ses sels, l'acycloguanosine et les 5 - (2 - halogénovinyl) - 2' - désoxyuridines, et dans laquelle le rapport molaire du premier composant actif au second composant actif est de 1:200 à 200:1.

2. Une composition selon la revendication 1, dans laquelle ledit second composant actif est l'acide phosphonoacétique ou un de ses sels.

3. Une composition selon la revendication 1, dans laquelle ledit second composant actif est l'acide phosphonoformique ou un de ses sels.

4. Une composition selon la revendication 1, dans laquelle ledit second composant actif est l'acycloguanoguanosine.

5. Une composition selon la revendication 1, dans laquelle ledit second composant actif est la bromovinyldésoxyuridine.

6. Une composition selon la revendication 1, dans laquelle ledit second composant actif est l'iodovinyl-désoxyuridine.

7. Une composition selon l'une quelconque des revendications précédentes, dans laquelle le rapport molaire des composants actifs est de 5:1 à 1:5.

8

8. Utilisation de deux composants actifs pour la fabrication d'un médicament pour le traitement des infections à virus d'herpès, dans laquelle les composants actifs sont combinés dans un rapport molaire de 1:200 à 200:1 et dans laquelle le premier composant actif est la 9 - [[2 - hydroxy - 1 - (hydroxyméthyl)éthoxy]méthyl]guanine et le second composant actif est choisi parmi l'acide phosphonoacétique et ses sels, l'acide phosphonoformique et ses sels, l'acycloguanosine et les 5 - (2 - halogénovinyl) - 2' - désoxyuridines.

9. Utilisation selon la revendication 8, dans laquelle le second composant actif est tel que défini dans l'une quelconque des revendications 2 à 6.

10. Utilisation selon la revendication 8 ou la revendication 9, dans laquelle le rapport molaire des composants actifs est de 5:1 à 1:5.

**Revendications pour l'Etat contractant: AT**

1. Un procédé pour préparer une composition thérapeutique douée d'activité contre les infections à virus d'herpès, qui consiste à combiner deux composants actifs dans un rapport molaire de 1:200 à 200:1, dans lequel le premier composant actif est la 9 - [[2 - hydroxy - 1 - (hydroxyméthyl)éthoxy]méthyl]guanine et le second composant actif est choisi parmi l'acide phosphonoacétique et ses sels, l'acide phosphonoformique et ses sels, l'acycloguanosine et les 5 - (2 - halogénovinyl) - 2' - désocyuridines.

2. Un procédé selon la revendication 1, dans lequel ledit second composant actif est l'acide phosphonoacétique ou un de ses sels.

3. Un procédé selon la revendication 1, dans lequel ledit second composant actif est l'acide phosphonoformique ou un de ses sels.

4. Un procédé selon la revendication 1, dans lequel ledit second composant actif est l'acycloguanoguanosine.

5. Un procédé selon la revendication 1, dans lequel ledit second composant actif est la bromovinyldésoxyuridine.

6. Un procédé selon la revendication 1, dans lequel ledit second composant actif est l'iodovinyl-désoxyuridine.

7. Un procédé selon l'une quelconque des revendications précédentes, dans lequel le rapport molaire des composants actifs est de 5:1 à 1:5.

8. Utilisation de deux composants actifs pour la fabrication d'un médicament pour le traitement des infections à virus d'herpès, dans laquelle les composants actifs sont combinés dans un rapport molaire de 1:200 à 200:1 et dans laquelle le premier composant actif est la 9 - [[2 - hydroxy - 1 - (hydroxyméthyl)éthoxy]méthyl]guanine et le second composant actif est choisi parmi l'acide phosphonoacétique et ses sels, l'acide phosphonoformique et ses sels, l'acycloguanosine et les 5 - (2 - halogénovinyl) - 2' - désoxyuridines.

9. Utilisation selon la revendication 8, dans laquelle le second composant actif est tel que défini dans l'une quelconque des revendications 2 à 6.

10. Utilisation selon la revendication 8 ou la revendication 9, dans laquelle le rapport molaire des composants actifs est de 5:1 à 1:5.